Europäisches Patentamt

European Patent Office

Office européen des brevets

(11) Numéro de publication: **0 474 946 A1**

## DEMANDE DE BREVET EUROPEEN

(21) Numéro de dépôt: 90402522.8

(22) Date de dépôt: 13.09.90

(51) Int. Cl.5: **C11C 3/00**, A61K 7/48, A61K 31/22, A61K 31/355, A61K 31/56

(43) Date de publication de la demande:
18.03.92 Bulletin 92/12

(84) Etats contractants désignés:
BE CH DE ES GB IT LI LU NL

(71) Demandeur: SARPAP
43, rue Maurice Ravel, B.P. 642
F-24106 Bergerac Cédex(FR)

(72) Inventeur: Bugat, Maurice
Le Grand Cros
F-24130 Gardonne(FR)

(74) Mandataire: Peuscet, Jacques et al
Cabinet Peuscet 68, rue d'Hauteville
F-75010 Paris(FR)

(54) Procédé de fabrication d'esters d'acides gras, produits isolés correspondants et compositions à usage humain ou vétérinaire les contenant.

(57) Procédé de fabrication d'esters d'acide(s) gras dans lequel on estérifie à l'aide d'au moins un acide gras en $C_4$ - $C_{24}$, un composé hydroxylé pris dans le groupe formé par les stérols, les tocophérols, les alcools terpéniques et les alcools aliphatiques non linéaires, qui sont présents sous forme libre et/ou ectérifiée dans les huiles ou corps gras naturels et font partie de la fraction insaponifiable de ces huiles ou corps gras naturels.

Esters gras obtenus sous forme isolée par ce procédé et compositions à usage humain ou vétérinaire contenant les produits obtenus dans un support approprié en quantité efficace.

La présente invention concerne un procédé de fabrication d'esters d'acides gras, les produits isolés obtenus par ce procédé et les compositions à usage humain ou vétérinaire les contenant.

Les huiles et les corps gras naturels sont principalement constitués par des triesters d'acide gras et de glycérol, mais contiennent également des constituants mineurs, dont des hydrocarbures et surtout des composés hydroxylés, autres que le glycérol, en majeure partie sous forme estérifiée par des acides gras et en faible quantité sous forme libre. L'ensemble des composés autres que les triglycérides contenu dans les huiles ou corps gras, est généralement désigné par le nom d'"insaponifiable" ou de "fraction insaponifiable". Cet insaponifiable représente généralement jusqu'à 1,5 % en poids de l'huile ou corps gras naturel, mais peut atteindre des teneurs plus élevées, par exemple 7 % dans les huiles d'avocats et les beurres de karité.

Les composés hydroxylés contenus dans l'insaponifiable sont des stérols, des alcools terpéniques, des tocophérols et des alcools aliphatiques.

La composition de l'insaponifiable est caractéristique d'une huile ou d'un corps gras déterminé. Cette composition est caractéristique dans son ensemble, c'est-à-dire que la nature, le nombre, les proportions relatives des composés présents dans chacune des catégories de composés (hydrocarbures, stérols, alcools terpéniques, tocophérols et alcools aliphatiques) sont caractéristiques. Elle est également caractéristique pour une catégorie de composés hydroxylés donnée (par exemple, les stérols) ; c'est-à-dire que le nombre et la nature des composés présents appartenant à cette catégorie, ainsi que leurs proportions relatives sont caractéristiques d'une huile ou d'un corps gras.

Les huiles et corps gras sont très largement utilisés en cosmétique et en pharmacie à usage humain ou vétérinaire et on a souvent émis l'hypothèse que leur action favorable était en grande partie due à la présence des composés hydroxylés, autres que le glycérol, sous forme estérifiée et/ou sous forme libre, et que l'action particulièrement favorable de certaines huiles ou corps gras était due à la composition particulière de l'insaponifiable.

On a donc cherché à utiliser l'insaponifiable, qui peut être séparé dans des procédés de traitement des huiles et corps gras naturels.

Selon l'un de ces procédés de traitement, on traite les triglycérides d'acides gras en chauffant les huiles ou corps gras avec une base alcaline, en particulier la potasse, et on extrait, d'une part, à l'aide d'une solution hydro-alcoolique, le glycérol et les acides gras saponifiés obtenus et, d'autre part l'insaponifiable, à l'aide d'un solvant, tel que l'hexane ou le chloroforme. Ce procédé relève d'une opération de "saponification", généralement mise en oeuvre en laboratoire.

Selon un autre procédé, on traite l'huile ou corps gras par entraînement à la vapeur et on sépare par décantation les composés entraînés de l'insaponifiable. Ce procédé, à caractéristique plus industrielle, est mis en oeuvre au cours du raffinage des huiles ou corps gras, et plus spécialement de la désodorisation. Dans ce procédé on entraîne, en plus des substances volatiles, des constituants qui constituent les condensats de désodorisation à partir desquels peuvent être isolés les insaponifiables.

Aucun des procédés connus de séparation des insaponifiables ne permet de recueillir les composés hydroxylés sous leur forme estérifiée. Les composés extraits sont sous forme libre, c'est-à-dire que le(s) groupement(s) fonctionnel(s) OH de ces constituants n'est (ne sont) pas combiné(s), et ils correspondent aux composés hydroxylés qui étaient sous forme libre dans l'huile ou corps gras, et/ou à ceux qui ont été obtenus par saponification des esters d'acide gras. Dans le cas du traitement par saponification, les composés hydroxylés sont obtenus en totalité à partir des esters. Par contre, l'entraînement à la vapeur ne permet de recueillir que la faible quantité de composé(s) hydroxylé(s) présent(s) sous forme libre dans l'huile ou corps gras.

On a utilisé, dans des compositions cosmétiques, ou dermatologiques , humaines ou vétérinaires , l'insaponifiable tel que séparé par l'un des procédés décrits ci-dessus, c'est-à-dire une fraction dans laquelle notamment les composés hydroxylés sont sous forme libre. Ces composés ont une action bénéfique, mais qui n'a généralement pas la nature et l'amplitude que l'on pouvait espérer au vu de l'action des huiles et corps gras naturels dont cet insaponifiable est extrait.

Le demandeur a émis l'hypothèse que, dans les huiles et corps gras naturels, l'activité cosmétique, pharmaceutique ou vétérinaire, des composés hydroxylés de l'insaponifiable serait en grande partie due au fait que ces composés hydroxylés sont en majeure partie sous forme estérifiée. Selon la présente invention, on a trouvé que les composés hydroxylés de l'insaponifiable, extraits des huiles et corps gras naturels sous forme libre et réestérifiés, présentaient l'activité attendue.

La présente invention concerne un procédé de fabrication d'esters gras par estérification à l'aide d'acides de différentes natures pris individuellement ou en mélange, des composés hydroxylés choisis parmi ceux qui font partie de l'insaponifiable d'huiles ou corps gras, pris isolément ou en mélange, ou par estérification de l'insaponifiable contenant les composés hydroxylés sous forme libre tels qu'extraits des huiles ou corps gras par un procédé de saponification ou d'entraînement à la vapeur par exemple.

Selon la présente invention, on reconstitue donc principalement par estérification ou réestérification des produits ou des mélanges de produits, qui sont contenus dans l'insaponifiable des huiles ou corps gras naturels de façon à les obtenir sous forme isolée, c'est-à-dire non mélangée à des triglycérides.

La présente invention a pour objet un procédé de fabrication d'esters d'acide(s) gras, selon lequel on fait réagir, à l'aide d'au moins un acide gras en $C_4$ - $C_{24}$ , au moins un composé hydroxylé pris dans le groupe formé par ceux des stérols, tocophérols, alcools terpéniques et alcools aliphatiques non linéaires, qui sont contenus, à l'état libre ou estérifié, dans les huiles ou corps gras naturel (le)s et qui font partie de la fraction insaponifiable desdites huiles ou corps gras.

L'estérification peut être effectuée par tout procédé connu. On peut, par exemple, faire réagir un acide gras avec un des composés hydroxylés sous forme d'acétate. La réaction est, en particulier, effectuée, à chaud, sous vide, en milieu protégé et en présence d'un catalyseur du type méthylate de sodium ou encore en présence d'amidure de sodium ou encore en présence de sodium métallique. Il est également possible d'effectuer une estérification directe du (des) composé(s) hydroxylé(s) par un (des) acide(s) gras, à chaud, sous vide, en milieu protégé, et en présence d'un catalyseur tel que l'acide paratoluène sulfonique. On effectue, de préférence, l'estérification en faisant réagir un chlorure d'acide gras avec un composé hydroxylé, à froid, à l'obscurité et en milieu protégé.

On peut utiliser tout acide gras : saturé ou non, pair ou impair, ramifié ou non. On utilise, de préférence, l'acide isostéarique qui a l'avantage de ne pas rancir, l'acide gamma linoléïque ou l'acide undécylénique qui a une action antifongique. Selon la présente invention, on utilise également avantageusement un mélange d'acides correspondant à celui qui est présent dans une huile ou corps gras déterminé(e). Par exemple, on peut utiliser un mélange en parties égales en poids d'acide palmitique, d'acide stéarique et d'acide oléique qui correspond au mélange d'acides gras contenu dans le beurre de cacao.

Selon un premier aspect de l'invention, on estérifie, de préférence, un mélange contenant des composés hydroxylés obtenu par traitement d'huile ou corps gras naturels et extraction au moins partielle de l'insaponifiable. Selon ce procédé, les composés hydroxylés de l'insaponifiable, obtenus sous forme libre après hydrolyse et/ou par extraction directe, sont réestérifiés de façon à reconstituer sous forme séparée, les esters des alcools autres que le glycérol qui sont contenus dans l'huile ou corps gras naturel.

Selon un premier procédé de traitement des huiles ou corps gras et d'extraction de l'insaponifiable, le mélange contenant les composés hydroxylés est obtenu par saponification d'une huile ou corps gras par une base alcaline, extraction d'une part du glycérol et des acides gras saponifiés dans une phase hydro-alcoolique et d'autre part, de la fraction insaponifiable dans un solvant et traitement de la fraction insaponifiable, en solution dans un solvant, par distillation du solvant.

Selon un second procédé, le traitement permettant d'obtenir le mélange contenant des composés hydroxylés, est un entraînement à la vapeur des huiles ou corps gras suivi d'une séparation par décantation de l'eau et de la fraction insaponifiable entraînée. En particulier, le mélange de composés hydroxylés provient de, l'entraînement à la vapeur réalisé dans les ateliers de désodorisation des unités de raffinage des huiles ou corps gras.

Selon un autre aspect de la présente invention, on estérifie des composés isolés ou des mélanges de composés isolés pris parmi les composés hydroxylés qui peuvent être contenus dans l'insaponifiable d'une huile ou corps gras.

Avantageusement, le composé hydroxylé est au moins un stérol pris dans le groupe formé par les phytostérols et les zoostérols. Parmi les phytostérols on peut citer le campéstérol, le stigmastérol, les alpha- et bêta-sitostérols et les Δ-7 avénastérols et parmi les zoostérols, le cholestérol et enfin les méthylstérols.

On utilise, en particulier, un mélange de stérols dont la composition correspond, en ce qui concerne le nombre et la nature des stérols utilisés ainsi que leurs proportions relatives, à celle de l'insaponifiable d'une huile ou corps gras déterminé(e). Par exemple, on peut reconstituer la composition en stérols de l'insaponifiable de l'huile d'amande douce, qui est la suivante en mg/1ØØ g d'huile :

| stigmastérol | 4 |
| campéstérol | 2 |
| bêta-sitostérol | 125 |

au total 131 mg pour 1ØØ g d'huile.

On peut utiliser tout tocophérol : qu'il soit sous forme alpha, bêta, gamma ou delta et quels que soient le nombre et la place des groupes méthyle. On utilise, de préférence, la vitamine E.

EP 0 474 946 A1

Le composé hydroxylé peut, selon l'invention, être au moins un alcool terpénique pris dans le groupe des alcools mono-, sesqui-, di-, et triterpéniques. Parmi les alcools terpéniques, on peut citer le phytol, le cycloarténol et les alpha- et bêta-amyrines. Comme dans le cas des stérols, on utilise préférentiellement un mélange d'alcools triterpéniques, dont la composition correspond, en ce qui concerne le nombre et la nature des alcools triterpéniques utilisés ainsi que leurs proportions relatives, à celle de la fraction insaponifiable d'une huile ou corps gras déterminé(e). Par exemple, on peut reconstituer la composition en alcools triterpéniques de l'huile de thé qui est la suivante en mg/100 g d'huile :

| alpha-amyrine | 25 |
| butyrospermol | 260 |
| cycloarténol | 70 |

On peut également utiliser un mélange de composés hydroxylés contenant des composés d'au moins deux des catégories suivantes : stérol, tocophérol et alcool terpénique.

Le mélange utilisé correspond, de préférence, à celui de l'insaponifiable d'une huile ou corps gras naturel déterminé. Par exemple, on peut reconstituer la composition en composés hydroxylés de l'huile de noisette qui est la suivante en mg/100 g d'huile :

| stérols | 180 |
| alcools terpéniques | 45 |
| tocophérols | 75 |

La présente invention a aussi pour objet les esters d'acide(s) gras obtenus sous forme isolée par le procédé de l'invention. Les produits selon la présente invention sont donc caractérisés par le fait qu'ils sont isolés. Ces produits sont en effet contenus dans les huiles ou corps gras naturel(le)s en mélange avec des triglycérides d'acide gras. Mais il n'était pas possible jusqu'à présent de les obtenir par un procédé d'extraction directe, en particulier par un procédé industriel d'extraction.

Ces esters d'acide(s) gras peuvent être commercialisés sous forme solide (poudre, paillettes par exemple) ou sous forme de solutions dans un solvant.

La présente invention a enfin pour objet une composition contenant au moins un ester d'acide(s) gras obtenu par le procédé selon l'invention, dans un support approprié en quantité efficace. Cette composition peut être notamment à usage humain ou vétérinaire. Elle est plus particulièrement utile en dermatologie. La quantité efficace susmentionnée est avantageusement comprise entre 0,01 % et 5 % en poids.

Le fait que l'on utilise, dans les compositions, les esters d'acide(s) gras de la présente invention, qui sont sous forme isolée, permet d'obtenir des compositions ayant une plus grande activité, que lorsqu'ils sont mélangés à une grande quantité de triglycérides et permet, de plus, de doser de façon plus précise leur activité.

L'exemple donné ci-dessous, à titre purement illustratif et non limitatif, permettra de mieux comprendre l'invention.

EXEMPLE

Cet exemple concerne la préparation d'une composition cosmétique à partir de l'huile de noisette.

1) Obtention de l'insaponifiable de l'huile de noisette.

On introduit 100 g d'huile de noisette dans un ballon muni d'un réfrigérant ascendant. On ajoute dans le ballon 1000 ml d'une solution de potasse dans l'éthanol à 12 % en poids. On chauffe et on maintient le mélange sous reflux pendant une heure. On ajoute ensuite 1000 ml d'eau par le haut du réfrigérant. La solution obtenue est extraite trois fois de suite à l'aide d'hexane comme solvant. On réunit les fractions obtenues de la solution dans l'hexane et an les lave trois fois de suite à l'aide de 1000 ml d'un mélange eau-alcool à 50 % d'alcool en poids.

On élimine l'hexane de la solution organique obtenue par distillation.

On obtient 300 mg d'un insaponifiable ayant la composition suivante en poids :

4

| stérols dont 90 % de bêta-sitostérol | 180 mg |
| alcool triterpénique dont 85 % de cycloarténol | 45 mg |
| tocophérols | 75 mg |

2) Estérification de l'insaponifiable

On utilise un ballon de 25 ml à trois tubulures, l'une constituant le passage d'un agitateur, la deuxième étant reliée à un réfrigérant ayant une garde au chlorure de calcium et la troisième à une ampoule à brome.

On dissout 200 mg de l'insaponifiable obtenu en 1 dans une quantité de chloroforme telle que le mélange obtenu soit homogène et on ajoute 80 mg de pyridine : les proportions sont de 1 mole de composé hydroxylé pour 2 moles de pyridine. On agite pour obtenir une solution homogène. On ajoute ensuite lentement 150 mg de chlorure d'acide palmitique dilué dans une quantité de chloroforme telle que le mélange obtenu soit homogène : les proportions sont de 1,1 mole de chlorure d'acide par mole de composé hydroxylé. On laisse reposer pendant une heure à température ambiante puis on chauffe pendant une heure au bain-marie. Ensuite, on ajoute 5 à 6 volumes d'eau et on maintient sous agitation vigoureuse. On ajoute de l'acide chlorhydrique à 10 % en poids, de façon à amener le pH à une valeur de 3 et on extrait en plusieurs fois à l'aide de chloroforme. On réunit les différents extraits obtenus et on les traite jusqu'à neutralité avec une solution de carbonate de sodium à 10 % en poids. On lave à l'eau et on sèche sur du sulfate de sodium.

On obtient 300 mg d'un produit cosmétique constitué par l'insaponifiable réestérifié ayant la composition suivante en mg :

| palmitate de stérols | 180 |
| palmitate d'alcools triterpéniques | 45 |
| palmitate de tocophérols | 75 |

3) Formulation

On prépare une crème ayant la formulation suivante :

| esters d'acide gras obtenu au stade 2 ci-dessus | 0,5 g |
| palmitate d'isopropyle | 5,0 g |
| huile de paraffine | 10,0 g |
| alcool cétylique | 2,0 g |
| produit émulsifiant vendu sous la dénomination commerciale "TWEEN 61" par la Société ICI | 5,0 g |
| conservateur | 0,2 g |
| oxyde de zinc | 0,5 g |
| eau   q.s.p. | 100 g |

Cette crème appliquée sur la peau du visage donne immédiatement une sensation de douceur et de confort. L'application régulière deux fois par jour de cette composition confère à l'épiderme de l'élasticité et une bonne hydratation nettement détectables après cinq jours.

**Revendications**

1. Procédé de fabrication d'esters d'acide(s) gras, caractérisé par le fait qu'on fait réagir au moins un acide gras en $C_4$- $C_{24}$, sur au moins un composé hydroxylé pris dans le groupe formé par ceux des stérols, tocophérols, alcools terpéniques et alcools aliphatiques non linéaires, qui sont contenus à l'état libre ou estérifié dans les huiles ou corps gras naturel(le)s et qui font partie de la fraction insaponifiable desdites huiles ou corps gras.

2. Procédé selon la revendication 1, caractérisé par le fait que le (ou les) acide(s) gras est (sont) pris dans le groupe formé par l'acide isostéarique, l'acide undécylénique et l'acide gamma-linoléique.

3. Procédé selon la revendication 1, caractérisé par le fait que l'on estérifie le (les) composé(s) hydroxylé-(s) à l'aide d'un mélange d'acides correspondant à celui qui est présent dans une huile ou corps gras déterminé(e).

4. Procédé selon l'une des revendications 1 à 3, caractérisé par le fait que l'on estérifie un mélange contenant des composés hydroxylés obtenu par traitement d'huile(s) ou corps gras et extraction au moins partielle de la fraction insaponifiable.

5. Procédé selon la revendication 4, caractérisé par le fait que le mélange contenant les composés hydroxylés est obtenu par saponification d'une huile ou corps gras par une base alcaline, extraction d'une part, dans une phase hydroalcoolique, du glycérol et des acides gras saponifiés et d'autre part, dans un solvant, de la fraction insaponifiable, et traitement de la fraction insaponifiable en solution dans le solvant par distillation.

6. Procédé selon la revendication 4, caractérisé par le fait que le traitement permettant d'obtenir le mélange contenant des composés hydroxylés est un entraînement à la vapeur des huiles ou corps gras suivi d'une séparation par décantation de l'eau et de la fraction insaponifiable entraînée.

7. Procédé selon l'une des revendications 1 à 4, caractérisé par le fait que le composé hydroxylé est au moins un stérol pris dans le groupe formé par les phytostérols et les zoostérols.

8. Procédé selon la revendication 7, caractérisé par le fait que l'on utilise un mélange de stérols, dont la composition correspond à celle du mélange de stérols présent dans la fraction insaponifiable d'une huile ou corps gras déterminé(e).

9. Procédé selon l'une des revendications 1 à 4, caractérisé par le fait que le composé hydroxylé est au moins un alcool terpénique pris dans le groupe des alcools mono-, sesqui-, di-, et triterpéniques.

10. Procédé selon la revendication 9, caractérisé par le fait que l'on utilise un mélange d'alcools terpéniques, dont la composition est celle du mélange d'alcools terpéniques présent dans la fraction insaponifiable d'une huile ou corps gras déterminé(e).

11. Procédé selon l'une des revendications 1 à 3, caractérisé par le fait que l'on utilise un mélange de composés hydroxylés dont l'ensemble des constituants appartient à au moins deux des catégories suivantes : stérols, alcools terpéniques et tocophérols.

12. Procédé selon la revendication 11, caractérisé par le fait que le mélange de composés hydroxylés correspond à celui de la fraction insaponifiable d'une huile ou corps gras déterminé(e).

13. Ester d'acide(s) gras obtenu sous forme isolée par le procédé selon l'une des revendications 1 à 12.

14. Composition à usage humain ou vétérinaire, caractérisée par le fait qu'elle contient, dans un support approprié, au moins un ester selon la revendication 13 en une quantité efficace.

15. Composition selon la revendication 14, caractérisée par le fait que la quantité efficace est comprise entre 0,01 et 5 % en poids.

16. Composition selon les revendications 13 ou 14 pour utilisation en cosmétique ou en pharmacie humaine ou vétérinaire.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | PATENT ABSTRACTS OF JAPAN, vol. 4, no. 190 (C-37)[672], 26 décembre 1980; & JP-A-55 130 919 (MORISHITA SEIYAKU K.K.) 11-10-1980 * Résumé * — — — | 1,4,6-8, 11-14,16 | C 11 C 3/00 A 61 K 7/48 A 61 K 31/22 A 61 K 31/355 A 61 K 31/56 |
| X | US-A-3 835 169   (ELISE KRAFT et al.) * Colonne 2, lignes 1-60,64-67; colonne 3, lignes 1-57; colonnes 3,4, exemples I-III; colonne 4, ligne 58 - colonne 5, ligne 10; colonne 5, ligne 40 - colonne 10, ligne 32 * — — — | 1,2,7,8, 13-16 | |
| X | AMERICAN PERFUMER AND COSMETICS, vol. 84, 1969, pages 37-40, US; M.J.D. VAN DAM: "New lanolin acid esters" * En entier * — — — | 1,3-5,7,8, 13,14,16 | |
| X | DERWENT FILE SUPPLIER WPI, 1977, accession no. 77-33710Y [19], Derwent Publications Ltd, Londres, GB; & JP-A-52 041 215 (EISAI K.K.) 30-03-1977 * Résumé * — — — | 13-16 | |
| A | US-A-4 101 562   (ASHOK KUMAR BHANDARI et al.) * Revendication 1 * — — — | 1 | DOMAINES TECHNIQUES RECHERCHES (Int. Cl.5) |
| A | FR-A-2 526 805   (EXSYMOL) * Revendications 1,10 * — — — — — | 1 | C 11 C A 61 K |

Le présent rapport de recherche a été établi pour toutes les revendications

| Lieu de la recherche | Date d'achèvement de la recherche | Examinateur |
|---|---|---|
| La Haye | 22 avril 91 | DEKEIREL M.J. |